# EUROPEAN PATENT APPLICATION

(11) **EP 1 857 051 A1**
(43) Date of publication of application: **21.11.2007**
(21) Application number: 07009060.0
(22) Date of filing: 04.05.2007
(51) Int. Cl.: A61B 8/00, A61B 8/08, A61B 8/13

(54) **An ultrasonic imaging apparatus and a method of displaying ultrasonic images**

(30) Priority: 09.05.2006 JP 2006130651
(71) Applicant: KABUSHIKI KAISHA TOSHIBA, Tokyo (JP); Toshiba Medical Systems Corporation, Otawara-shi Tochigi (JP)
(72) Inventor: Akaki, Kazuya, Otawara-shi Tochigi (JP); Kurita, Koichiro, Otawara-shi Tochigi (JP); Gunji, Takayuki, Otawara-shi Tochigi (JP); Nakajima, Osamu, Otawara-shi Tochigi (JP); Higuchi, Jiro, Otawara-shi Tochigi (JP)
(74) Representative: Kramer - Barske - Schmidtchen

(57) **Abstract**

An ultrasonic probe transmits ultrasonic waves to a subject to be examined and receives reflected waves from the subject to be examined. An image processor generates three-dimensional image data based on the reflected waves received by the ultrasonic probe. Moreover, the image processor displays a mark indicating the positional relationship between the three-dimensional image and the ultrasonic probe on a display, the mark overlapping the three-dimensional image based on the three-dimensional image data.

## Description

### BACKGROUND OF THE INVENTION

### 1. Field of the Invention

The present invention relates to an ultrasonic imaging apparatus for obtaining and displaying three-dimensional images and a method of displaying ultrasonic images. More particularly, the present invention relates to a technology for improving the operability of ultrasonic probes.

### 2. Description of the Related Art

An ultrasonic imaging apparatus capable of obtaining and displaying a three-dimensional image can rotate, move, or change the orientation of the three-dimensional image displayed on a display by means of instructions given by an operator while an ultrasonic probe is fixed on a subject to be examined. In order to display a desired three-dimensional image on the display, the operator is required to move or rotate the ultrasonic probe on the subject to be examined. However, it is difficult for the operator to ascertain the positional relationship between the three-dimensional image displayed on the display and the ultrasonic probe, so there is a problem in that it is hard to know which direction the ultrasonic probe should be moved or rotated on the subject to be examined.

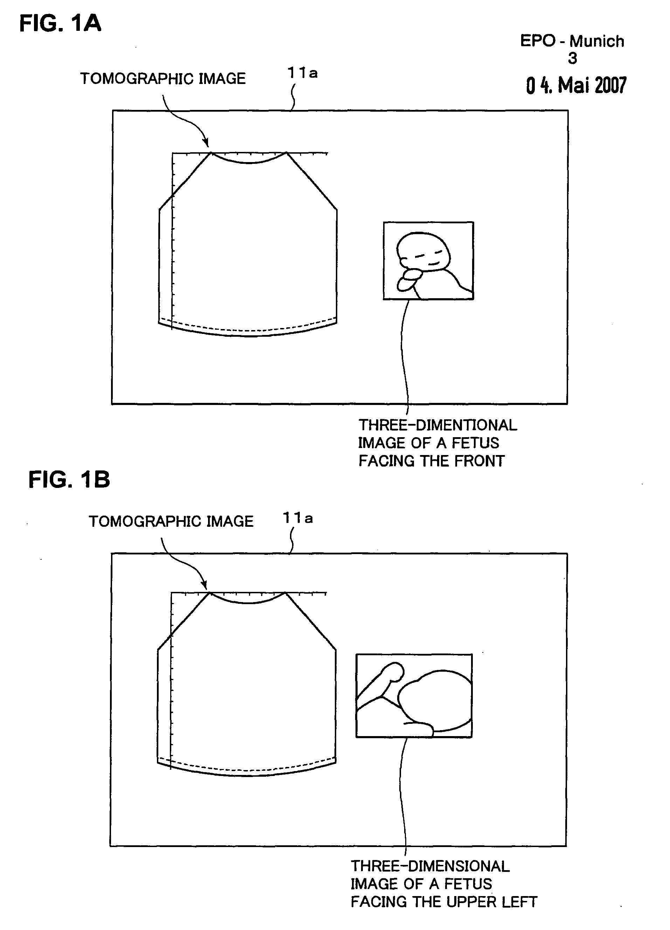

For example, the case of obtaining a three-dimensional image of a fetus and displaying the three-dimensional image on the display is described with reference to Fig. 1A and Fig. 1B. Fig. 1A and Fig. 1B are views of a screen showing a three-dimensional image obtained by a conventional ultrasonic imaging apparatus. The ultrasonic imaging apparatus obtains a three-dimensional image of a fetus and displays the three-dimensional image of the fetus on a screen 11 a of the display as shown in Fig. 1 A. Incidentally, in the examples shown in Fig. 1A and 1 B, a tomographic image is displayed on the display along with the three-dimensional image. In the example shown in Fig. 1A, the three-dimensional image of the fetus is directed to the front of the screen 11a. Then, when the operator gives instructions to rotate the three-dimensional image, it is possible to display the three-dimensional image of the fetus such that it is facing the upper left of the screen 11 a as shown in Fig. 1B. This operation enables the left side of the body of the fetus to be easily seen. However, it is difficult for the operator to ascertain the positional relationship between the three-dimensional image displayed on the display and the ultrasonic probe. Therefore, when observing the abdomen of the fetus in this state, it becomes hard to know which direction the ultrasonic probe should be moved or rotated on the subject to be examined.

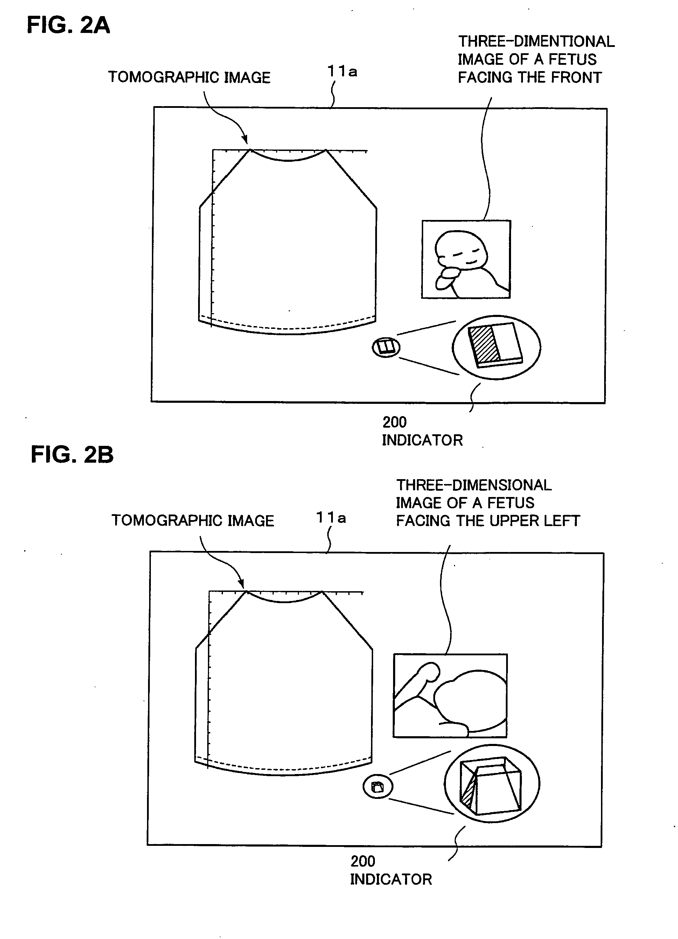

Therefore, the conventional ultrasonic imaging apparatus displays on the display a frame indicating relatively the same orientation as the three-dimensional image displayed on the display, and uses the frame as an indicator representing the orientation of the three-dimensional image. Herein, examples of the indicator are described with reference to Fig. 2A and Fig. 2B. Fig. 2A and Fig. 2B are views of a screen showing a three-dimensional image and an indicator obtained by a conventional ultrasonic imaging apparatus. For example, as shown in Fig. 2A, when a three-dimensional image of a fetus is displayed on the screen 11 a of the display such that it is facing the front, an indicator 200 that is a box-shape frame is displayed on the screen 11 a in accordance with the orientation of the three-dimensional image of the fetus. Then, when directing the orientation of the three-dimensional image of the fetus to the upper left as shown in Fig. 2B by rotating the three-dimensional image on the screen according to the instructions given by the operator, the orientation of the indicator 200 is also rotated in accordance with the orientation of the three-dimensional image of the fetus and is displayed on the screen 11 a. In this way, by displaying the indicator 200 that is directed in the same direction as the three-dimensional image on the screen 11a, the operator observes the indicator to analogize the orientation of the three-dimensional image.

However, when displaying the indicator 200 on the screen 11 a of the display as shown in Fig. 2A and Fig. 2B, the operator is required to relatively analogize the orientation of the three-dimensional image, based on the orientation of the indicator 200. Therefore, it was difficult to intuitively ascertain the orientation of the three-dimensional image.

In addition, even when the indicator 200 indicating the same direction as the three-dimensional image is displayed on the screen 11a of the display, it was difficult to intuitively ascertain the relative positional relationship between the ultrasonic probe and the three-dimensional image. Consequently, it was hard to know which direction the ultrasonic probe should be moved or rotated in order to display the desired image.

### SUMMARY OF THE INVENTION

The present invention is intended to provide an ultrasonic imaging apparatus that is capable of easily ascertaining the relative positional relationship between a three-dimensional image displayed on a display and an ultrasonic probe, and a method of displaying ultrasonic images.

The first embodiment of the present invention is an ultrasonic imaging apparatus comprising an ultrasonic probe transmitting ultrasonic waves on a subject to be examined and receiving the reflected waves from said subject to be examined, and an image processor generating three-dimensional image data based on the reflected waves received by said ultrasonic probe, and displaying a mark indicating the positional relationship between the three-dimensional image and said ultrasonic probe on a display, said mark overlapping said three-dimensional image based on said three-dimensional image data.

According to the first embodiment, the mark indicating the positional relationship between the three-dimensional image and the ultrasonic probe is displayed on the display, the mark overlapping the three-dimensional image; therefore, referencing the mark enables the operator to easily ascertain the relative positional relationship between the ultrasonic probe and the three-dimensional image.

In addition, the second embodiment of the present invention is an ultrasonic imaging apparatus according to the first embodiment, wherein the image processor adds the mark indicating the positional relationship between the three-dimensional image and the ultrasonic probe to the three-dimensional image data, and displays, on the display, a three-dimensional image based on the three-dimensional image data to which the mark has been added.

According to the second embodiment, adding the mark indicating the positional relationship with the ultrasonic probe to the three-dimensional image data displays the mark on the three-dimensional image. Referencing this mark enables the relative positional relationship between the ultrasonic probe and the three-dimensional image to be easily ascertained.

In addition, the third embodiment of the present invention is an ultrasonic imaging apparatus comprising an ultrasonic probe transmitting ultrasonic waves on a subject to be examined and receiving the reflected waves from the subject to be examined, a tomographic image data generator generating a plurality of tomographic image data along a predetermined direction based on the reflected waves received by the ultrasonic probe, a mark forming part writing a predetermined mark into tomographic image data obtained at a predefined position in the predetermined direction among the plurality of tomographic image data along the predetermined direction, a three-dimensional image data generator generating three-dimensional image data based on the plurality of tomographic image data including the tomographic image data into which the predetermined mark has been written, and a display for displaying a three-dimensional image based on the three-dimensional image data.

According to the third embodiment, writing the mark into the tomographic image data obtained at the predefined position among the plurality of tomographic image data and generating three-dimensional image data based on the plurality of tomographic image data displays the mark on the three-dimensional image, which is at the position corresponding to the predefined position described above. Referencing this mark enables the relative positional relationship between the ultrasonic probe and the three-dimensional image to be easily ascertained.

In addition, the fourth embodiment of the present invention is an ultrasonic imaging apparatus comprising an ultrasonic probe transmitting ultrasonic waves on a subject to be examined and receiving the reflected waves from the subject to be examined, a tomographic image data generator generating a plurality of tomographic image data along a predetermined direction based on the reflected waves received by the ultrasonic probe, a mark forming part writing a predetermined mark into a predetermined range of each tomographic image data for the plurality of tomographic image data along the predetermined direction, a three-dimensional image data generator generating three-dimensional image data based on the plurality of tomographic image data into which the predetermined mark has been written, and a display for displaying a three-dimensional image based on the three-dimensional image data.

In addition, the fifth embodiment of the present invention is a method of displaying ultrasonic images comprising: transmitting ultrasonic waves on a subject to be examined and receiving the reflected waves from the subject to be examined using an ultrasonic probe, generating three-dimensional image data based on the reflected waves received by the ultrasonic probe, displaying a mark indicating the positional relationship between the three-dimensional image and the ultrasonic probe on a display, the mark overlapping the three-dimensional image based on the three-dimensional image data.

In addition, the sixth embodiment of the present invention is a method of displaying ultrasonic images comprising: transmitting ultrasonic waves on a subject to be examined and receiving the reflected waves from the subject to be examined using an ultrasonic probe, generating a plurality of tomographic image data along a predetermined direction based on the reflected waves received by the ultrasonic probe, writing a predetermined mark into tomographic image data obtained at a predefined position in the predetermined direction among the plurality of tomographic image data along the predetermined direction, generating three-dimensional image data based on the plurality of tomographic image data including the tomographic image data into which the predetermined mark has been written, and displaying a three-dimensional image based on the three-dimensional image data on a display.

In addition, the seventh embodiment of the present invention is a method of displaying ultrasonic images comprising: transmitting ultrasonic waves on a subject to be examined and receiving the reflected waves from the subject to be examined using an ultrasonic probe, generating a plurality of tomographic image data along a predetermined direction based on the reflected waves received by the ultrasonic probe, writing a predetermined mark into a predetermined range of each tomographic image data for the plurality of tomographic image data along the predetermined direction, generating three-dimensional image data based on the plurality of tomographic image data into which the predetermined mark has been written, and displaying a three-dimensional image based on the three-dimensional image data on a display.

### BRIEF DESCRIPTION OF THE DRAWINGS

Fig. 1A is a view of a screen showing a three-dimensional image obtained by a conventional ultrasonic imaging apparatus.
Fig. 1B is a view of a screen showing a three-dimensional image obtained by a conventional ultrasonic imaging apparatus.
Fig. 2A is a view of a screen showing a three-dimensional image and an indicator obtained by a conventional ultrasonic imaging apparatus.
Fig. 2B is a view of a screen showing a three-dimensional image and an indicator obtained by a conventional ultrasonic imaging apparatus.
Fig. 3 is a block diagram showing the ultrasonic imaging apparatus according to an embodiment of the present invention.
Fig. 4A is a perspective view of the ultrasonic probe according to an embodiment of the present invention.
Fig. 4B is a front view of the ultrasonic probe according to an embodiment of the present invention.
Fig. 5A is a view of the processing for displaying a mark overlapping a three-dimensional image and a schematic drawing representing a tomographic image.
Fig. 5B is a view of the processing for displaying a mark overlapping a three-dimensional image and a schematic drawing representing a tomographic image.
Fig. 6A is a view of the processing for displaying a mark overlapping a three-dimensional image and a schematic drawing representing a tomographic image.
Fig. 6B is a view of the processing for displaying a mark overlapping a three-dimensional image and a schematic drawing representing a tomographic image.
Fig. 7A is a view of the processing for displaying a mark overlapping a three-dimensional image and a schematic drawing representing a tomographic image.
Fig. 7B is a view of the processing for displaying a mark overlapping a three-dimensional image and a schematic drawing representing a tomographic image.
Fig. 7C is a view of the processing for displaying a mark overlapping a three-dimensional image and a schematic drawing representing a tomographic image.
Fig. 7D is a view of the processing for displaying a mark overlapping a three-dimensional image and a schematic drawing representing a tomographic image.
Fig. 8A is a view of the processing for displaying a mark overlapping a three-dimensional image and a schematic drawing representing a tomographic image.
Fig. 8B is a view of the processing for displaying a mark overlapping a three-dimensional image and a schematic drawing representing a tomographic image.
Fig. 8C is a view of the processing for displaying a mark overlapping a three-dimensional image and a schematic drawing representing a tomographic image.
Fig. 8D is a view of the processing for displaying a mark overlapping a three-dimensional image and a schematic drawing representing a tomographic image.
Fig. 9 is a flow chart showing a series of operations by the ultrasonic imaging apparatus according to an embodiment of the present invention.
Fig. 10A is a view of a screen showing a three-dimensional image obtained by the ultrasonic imaging apparatus according to an embodiment of the present invention.
Fig. 10B is a view of a screen showing a three-dimensional image obtained by the ultrasonic imaging apparatus according to an embodiment of the present invention.

### DETAILED DESCRIPTION OF THE EMBODIMENTS

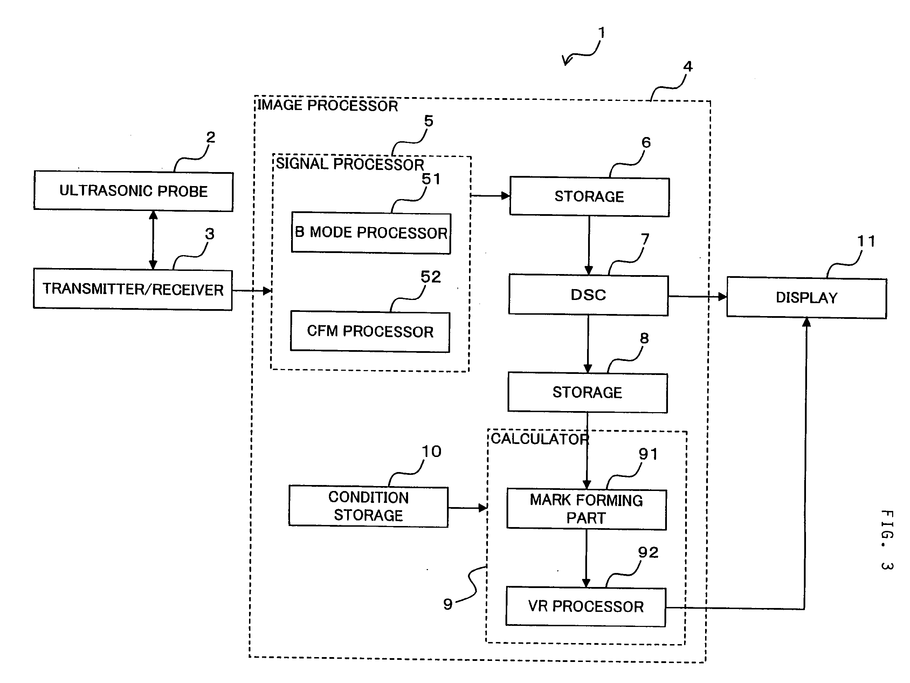

The configuration of an ultrasonic imaging apparatus according to an embodiment of the present invention is described with reference to Fig. 3. Fig. 3 is a block diagram showing the ultrasonic imaging apparatus according to an embodiment of the present invention.

The ultrasonic imaging apparatus 1 according to the present embodiment is configured to comprise an ultrasonic probe 2, a transmitter/receiver 3, an image processor 4, and a display 11.

For the ultrasonic probe 2, a two-dimensional array probe on which a plurality of ultrasonic transducers are two-dimensionally arranged, or a one-dimensional array probe on which a plurality of ultrasonic transducers are arranged in a predetermined direction (scanning direction) is employed. The two-dimensional array probe has a plurality of ultrasonic transducers that are two-dimensionally arranged, so it can three-dimensionally transmit ultrasonic waves and can receive three-dimensional data as an echo signal. In addition, the one-dimensional array probe can receive three-dimensional data as an echo signal by mechanically swinging the ultrasonic transducers in the direction perpendicular to the scanning direction. In the present embodiment, a one-dimensional array probe may be employed, or a two-dimensional array probe may be employed.

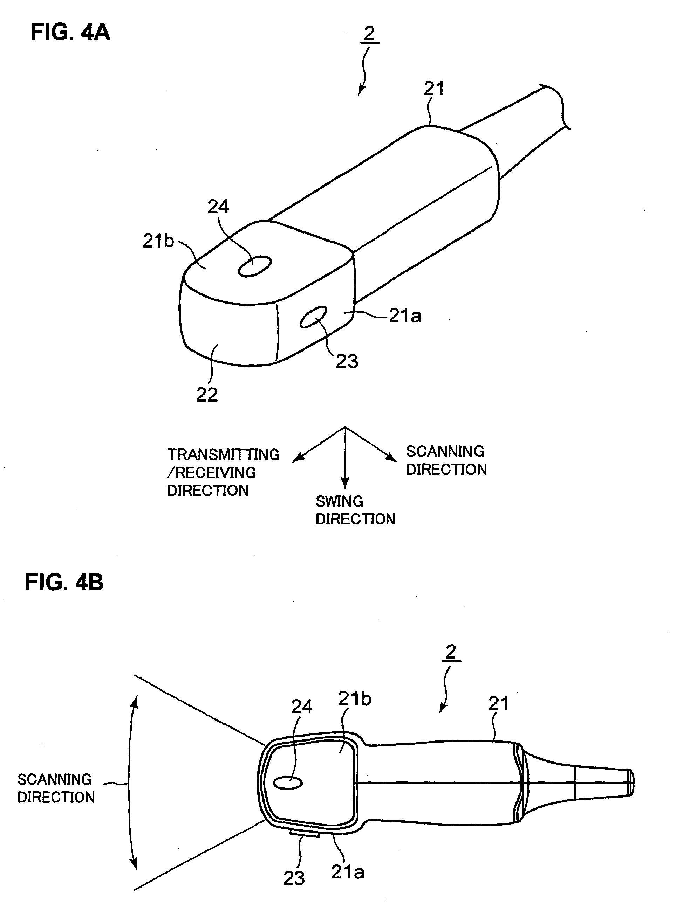

Herein, the appearance of the ultrasonic probe 2 is described with reference to Fig. 4A and Fig. 4B. Fig. 4A is a perspective view of the ultrasonic probe according to an embodiment of the present invention. Fig. 4B is a front view of the ultrasonic probe according to an embodiment of the present invention. Herein, the case in which a one-dimensional array probe is employed as the ultrasonic probe 2 is described.

As shown in Fig. 4A and Fig. 4B, a first physical mark 23 and a second physical mark 24 are provided on the surface of a case 21 of the ultrasonic probe 2. The case 21 has four side surfaces. The first physical mark 23 is provided in the center of a first side surface 21 a. The second physical mark 24 is provided in the center of a second surface 21b. The first physical mark 23 and the second physical mark 24 have morphologies such as a quadrangle, a circle, or an oval, and are formed as a depressed or raised protruding shape. The operator can recognize the first physical mark 23 and the second physical mark 24 by forming the first physical mark 23 and the second physical mark 24 as either a depressed or protruding shape.

A transmitting/receiving surface 22 is in contact with the body surface of a subject to be examined. A plurality of ultrasonic transducers is provided inside the case 21. The plurality of ultrasonic transducers is arranged in a line in the scanning direction on the one-dimensional array probe.

As shown in Fig. 4B, the second side surface 21b is a side surface parallel to the scanning direction for scanning ultrasonic waves. The first side surface 21a is a side surface parallel to the direction perpendicular to the scanning direction.

For example, when transmitting/receiving ultrasonic waves while swinging ultrasonic transducers in the direction perpendicular to the scanning direction (hereinafter, may be referred to as the "swing direction"), the first physical mark 23 is formed at the center of the swing direction. In addition, the second physical mark 24 is formed at the center of the scanning direction.

Incidentally, in the present embodiment, the first physical mark 23 is provided in the center of the first surface 21a. As another example, the first physical mark 23 may be provided on the end part of the first side surface 21a. Consequently, the first physical mark 23 is to be provided on the end part in the swing direction. In addition, in the present embodiment, the second physical mark 24 is provided in the center of the second side surface 21b. As another example, the second physical mark 24 may be provided on the end part of the second side surface 21b. Consequently, the second physical mark 24 is to be provided on the end part in the scanning direction. In addition, the first physical mark 23 and the second physical mark 24 may be provided on a part other than the center or the end part.

In the present embodiment, the case of employing a one-dimensional array probe as the ultrasonic probe 2 and swinging ultrasonic transducers in the direction perpendicular to the scanning direction (swing direction) to scan a three-dimensional region is described. A plurality tomographic image data along the swing direction is obtained by transmitting/receiving ultrasonic waves while swinging the ultrasonic transducers in this way.

The transmitter/receiver 3 is provided with a transmitting part and a receiving part. The transmitting part generates ultrasonic waves by supplying electrical signals to the ultrasonic probe 2. The receiving part receives echo signals received by the ultrasonic probe 2. The signals received by the transmitter/receiver 3 are output to the signal processor 5 of the image processor 4.

The signal processor 5 is configured to comprise a B-mode processor 51 and a CFM processor 52.

The B-mode processor 51 converts the amplitude information of the echo to an image and generates B-mode ultrasonic raster data from the echo signals. The CFM processor 52 converts the moving bloodstream information to an image and generates color ultrasonic raster data. The storage 6 temporarily stores the ultrasonic raster data generated by the signal processor 5.

A DSC (Digital Scan Converter) 7 converts the ultrasonic raster data into image data represented by Cartesian coordinates in order to obtain an image represented by a Cartesian coordinate system (scan conversion processing). Then, the image data is output from the DSC 7 to the display 11, and an image based on the image data is displayed on the display 11. For example, the DSC 7 generates tomographic image data as two-dimensional information based on the B-mode ultrasonic raster data, and outputs the tomographic image data to the display 11. The display 11 displays a tomographic image based on the tomographic image data. Incidentally, the signal processor 5 and the DSC 7 are one example of the "tomographic image data generator" of the present invention.

In the present embodiment, image data such as the tomographic image data output from the DSC 7 is output to and stored on the storage 8. In the present embodiment, a plurality of tomographic image data along the swing direction is obtained and is stored on the storage 8.

A calculator 9 reads image data from the storage 8, and generates three-dimensional image data based on the image data. In the present embodiment, the calculator 9 reads a plurality of tomographic image data along the swing direction from the storage 8, and generates three-dimensional image data based on the plurality of tomographic image data. Moreover, the calculator 9 writes a mark for indicating the orientation of the ultrasonic probe 2 into a predetermined position in the three-dimensional image. Hereinafter, the configuration and processing content of this calculator 9 are described. Incidentally, although a fetus is described as the subject of radiography in the present embodiment, an organ such as the heart may be the subject of radiography.

When a plurality of tomographic image data along the swing direction is obtained by the ultrasonic probe 2 and is stored on the storage 8, the calculator 9 reads the plurality of tomographic image data from the storage 8.

The mark forming part 91 selects tomographic image data obtained at a predetermined position in the swing direction among a plurality of tomographic image data along the swing direction, and writes a predetermined mark into the selected tomographic image data. This predetermined position is a position predefined by the operator. This predetermined position is a position recognized by the operator. For example, the mark forming part 91 selects tomographic image data obtained at the center of the swing direction among a plurality of tomographic image data obtained along the swing direction, and writes a predetermined mark into the tomographic image data at the center. Information indicating the position at which the mark forming part 91 selects tomographic image data, information indicating the position into which a mark is written, and information regarding the mark is pre-stored in a condition storage 10. In addition, the operator can use an operating part (not shown) to optionally change the position at which tomographic image data is selected or the position into which a mark is written. For example, a position at the end part in the swing direction may be optionally designated as well as the center of the swing direction.

For example, the first physical mark 23 is provided in the center of the swing direction of the case 21 and a mark is written into tomographic image data obtained at the center of the swing direction by the mark forming part 91. As a result, the position of the first physical mark 23 and the position in the swing direction of the tomographic image data into which a mark has been written correspond with each other.

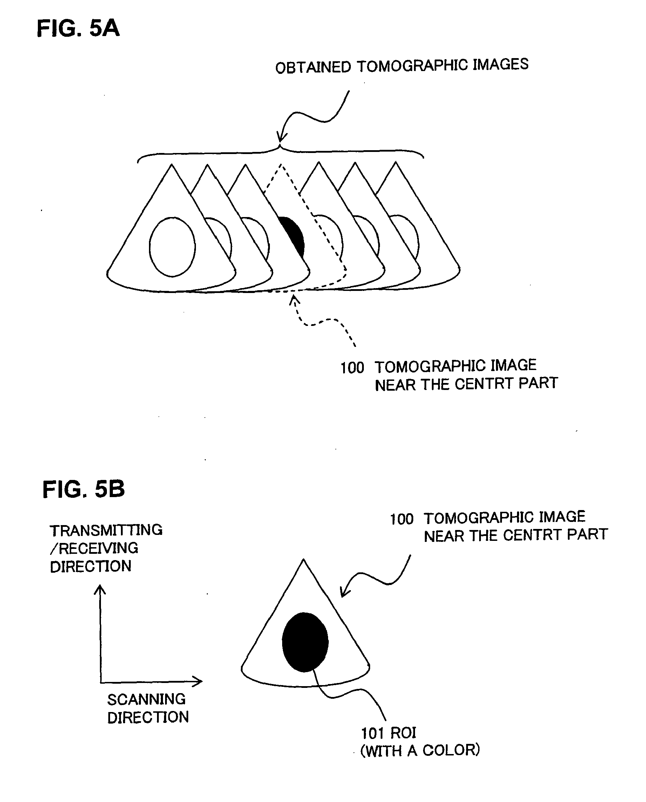

Herein, processing for forming a mark by the mark forming part 91 is described with reference to Fig. 5A and Fig. 5B. Fig. 5A and Fig. 5B are views of the processing for displaying a mark overlapping a three-dimensional image and a schematic drawing representing a tomographic image. As shown in Fig. 5A, for example, the mark forming part 91 selects tomographic image data 100 obtained at the center of the swing direction among a plurality of tomographic image data obtained along the swing direction. Then, the mark forming part 91 writes a predetermined mark into the selected tomographic image data 100. As shown in Fig. 5B for example, the mark forming part 91 colors images included in a preset ROI (Region Of Interest) 101 with a preset color for the tomographic image data 100 obtained at the center of the swing direction. Incidentally, information regarding the ROI 101 (e.g., information indicating the size or the position of the ROI 100) and information indicating colors are pre-stored in the condition storage 10.

Then, the mark forming part 91 outputs a plurality of tomographic image data read from the storage 8 along with the colored tomographic image data to a VR processor 92. In the present embodiment, it is intended to obtain an image of a fetus; therefore, the ROI 101 is set so as to include the image of the fetus. The operator can optionally set this ROI 101.

The VR processor 92 receives a plurality of tomographic image data from the mark forming part 91, and generates volume data based on the plurality of tomographic image data. Then, the VR processor 92 applies volume rendering on the volume data to generate image data as three-dimensional information (hereinafter, may be referred to as "VR image data"). The VR processor 92 outputs the VR image data to the display 11. The display 11 displays a VR image based on the VR image data (three-dimensional image) on the screen. Incidentally, the VR processor 92 is one example of the "three-dimensional image data generator" of the present invention.

As described above, a mark is written into predetermined tomographic image data. Furthermore, three-dimensional image data is generated based on a plurality of tomographic image data including the tomographic image data. As a result, a display mark corresponding to the mark written into the predetermined tomographic image data is displayed on the VR image displayed on the display 11.

A mark is written into the tomographic image data obtained at the center of the swing direction and the first physical mark 23 is provided in the center of the swing direction of the case 21. Therefore, the display mark on the VR image displayed on the display 11 corresponds with the first physical mark 23. The display mark on the VR image displayed on the display 11 and the first physical mark 23 provided on the case 21 of the ultrasonic probe 2 correspond with each other. Therefore, referencing the orientation of the display mark on the VR image and the orientation of the first physical mark 23 enables the operator to easily determine in which direction the ultrasonic probe 2 should be moved or rotated in order to obtain the desired image. That is, it enables the relative positional relationship between the ultrasonic probe 2 and the three-dimensional image to be easily ascertained.

Incidentally, when the first physical mark 23 is provided on the end part in the swing direction of the case 21, the mark forming part 91 writes a mark into tomographic image data obtained at the end part in the swing direction so that it corresponds with the position of the first physical mark 23. Consequently, the display mark on the VR image and the first physical mark 23 provided on the case 21 of the ultrasonic probe 2 correspond with each other. This enables the relative positional relationship between the ultrasonic probe 2 and the three-dimensional image to be easily ascertained.

In addition, marks formed by the mark forming part 91 are not limited to the examples shown in Fig. 5A and Fig. 5B. Hereinafter, other examples of forming a mark by the mark forming part 91 are described.

### Example of Modification 1

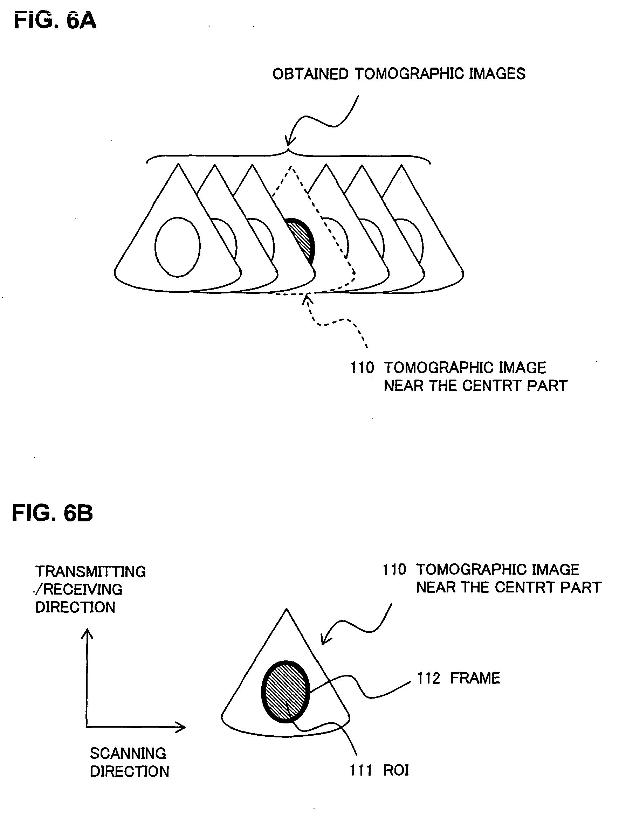

First, Example of Modification 1 is described with reference to Fig. 6A and Fig. 6B. Fig. 6A and Fig. 6B are views of the processing for displaying a mark overlapping a three-dimensional image and a schematic drawing representing a tomographic image. For example, as shown in Fig. 6A, the mark forming part 91 selects tomographic image data 110 obtained at the center of the swing direction among a plurality of tomographic image data obtained along the swing direction. Then, as shown in Fig. 6B, the mark forming part 91 writes a frame 112 surrounding a preset ROI 111 as a mark into the tomographic image data 110 obtained at the center of the swing direction. For example, the mark forming part 91 colors the frame 112 or increases the pixel value thereof to be higher than that of the surrounding area. Information regarding the ROI 111 and information regarding the frame 112 are pre-stored in the condition storage 10. Then, the mark forming part 91 outputs a plurality of tomographic image data read from the storage 8 along with the tomographic image data into which the frame (mark) 112 has been written to the VR processor 92.

The VR processor 92 receives the plurality of tomographic image data from the mark forming part 91, and applies volume rendering to generate the VR image data. The display 11 displays a VR image based on the VR image data (three-dimensional image) on the screen.

As described above, a mark is written into a predetermined tomographic image data and three-dimensional image data is generated based on a plurality of tomographic image data including the tomographic image data. As a result, a display mark corresponding to the mark written into the predetermined tomographic image data is displayed on the VR image displayed on the display 11.

Since the frame (mark) 112 is written into the tomographic image data obtained at the center of the swing direction and the first physical mark 23 is provided at the center of the swing direction of the case 21, the display mark on the VR image displayed on the display 11 corresponds with the first physical mark 23. Consequently, referencing the orientation of the display mark on the VR image and the orientation of the first physical mark 23 enables the operator to easily ascertain the relative positional relationship between the ultrasonic probe 2 and the three-dimensional image.

Incidentally, when the first physical mark 23 is provided on the end part in the swing direction of the case 21, the mark forming part 91 writes the frame (mark) 112 into tomographic image data obtained at the end part in the swing direction so that it corresponds with the position of the first physical mark 23. Consequently, the display mark on the VR image and the first physical mark 23 provided on the case 21 of the ultrasonic probe 2 correspond with each other. This enables the relative positional relationship between the ultrasonic probe 2 and the three-dimensional image to be easily ascertained.

In this Example of Modification 1, the mark forming part 91 writes the frame 112 surrounding the ROI 111 as a mark into the tomographic image data 110 so that a display mark is displayed on the VR image.

Besides the manner of writing the mark into tomographic image data as described above, the calculator 9 may detect an outline of the ROI 111 from the tomographic image data 110, and display, on the display 11, a display mark representing the outline of the ROI 111 overlapping the VR image.

For example, the calculator 9 selects tomographic image data 110 obtained at the center of the swing direction among a plurality of tomographic image data obtained along the swing direction. Then, the calculator 9 detects an outline of the ROI 111 from the tomographic image data 110, and generates a display mark representing the outline. Moreover, the calculator 9 reads a plurality of tomographic image data from the storage 8, and applies volume rendering to generate the VR image data. Unlike the above processing, no mark is written into this VR image data.

Then, the calculator 9 displays a VR image based on the VR image data on the display 11. Moreover, the calculator 9 displays, on the display 11, a display mark representing the outline of the ROI 111 overlapping the position (coordinates) at which the ROI 111 has been detected in the VR image.

The display mark that is displayed overlapping the VR image corresponds with the first physical mark 23, and thus, referencing the orientation of the display mark on the VR image and the orientation of the first physical mark 23 enables the operator to easily ascertain the relative positional relationship between the ultrasonic probe 2 and the three-dimensional image.

### Example of Modification 2

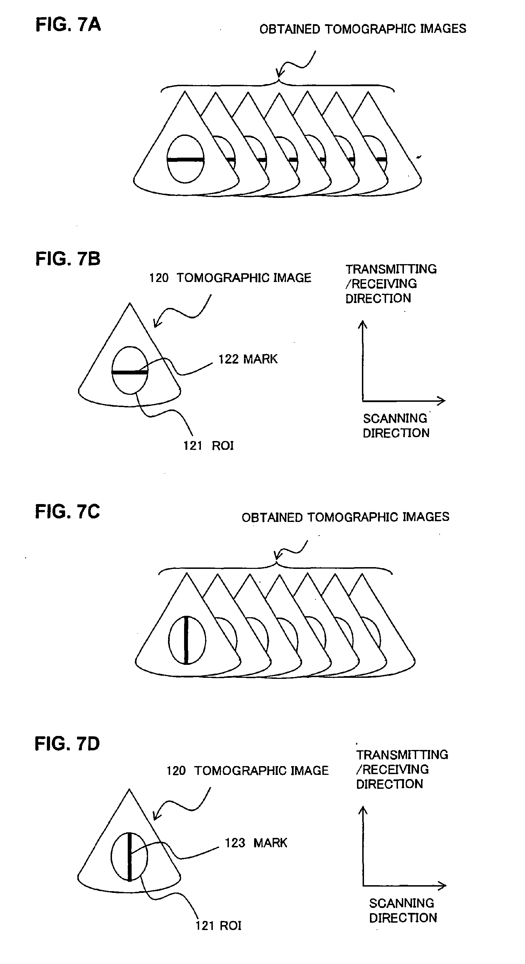

Next, Example of Modification 2 is described with reference to Fig. 7A, Fig. 7B, Fig. 7C, and Fig. 7D. Fig. 7A, Fig. 7B, Fig. 7C, and Fig. 7D are views of the processing for displaying a mark overlapping a three-dimensional image and a schematic drawing representing a tomographic image. As shown in Fig. 7A, the mark forming part 91 writes a mark into all tomographic image data obtained along the swing direction. As shown in Fig. 7B, for example, the mark forming part 91 writes a straight mark 122 crossing in the scanning direction (transverse direction) at the center of a preset ROI 121 into all tomographic image data 120. This straight mark 122 is written along the scanning direction. For example, the mark forming part 91 colors the straight mark 122 or increases the pixel value thereof to be higher than that of the surrounding area. Information regarding the ROI 121 and information regarding the straight mark 122 are pre-stored in the condition storage 10. Then, the mark forming part 91 outputs all the tomographic image data into which the straight mark 122 has been written to the VR processor 92.

In addition, as shown in Fig. 7C and Fig. 7D, the mark forming part 91 may also write a straight mark 123 expanding in the transmitting/receiving direction (longitudinal direction) at the center of the scanning direction of a preset ROI 121 into all tomographic image data 120. This straight mark 123 is written along the transmitting/receiving direction of ultrasonic waves. For example, the mark forming part 91 colors the straight mark 123 or increases the pixel value thereof to be higher than that of the surrounding area. Information regarding the straight mark 123 is pre-stored in the condition storage 10. Then, the mark forming part 91 outputs all the tomographic image data into which the straight mark 123 has been written to the VR processor 92.

The VR processor 92 receives the plurality of tomographic image data from the mark forming part 91, and applies volume rendering to generate the VR image data. The display 11 displays a VR image based on the VR image data (three-dimensional image) on the screen.

As described above, a mark is written into a plurality of tomographic image data and VR image data is generated based on the plurality of tomographic image data. As a result, a display mark corresponding to the mark is displayed on the VR image displayed on the display 11.

Since the straight mark 123 is written into the center of the scanning direction and the second physical mark 24 is provided in the center of the scanning direction of the case 21, the display mark on the VR image displayed on the display 11 corresponds with the second physical mark 24. Since the display mark on the VR image displayed on the display 11 and the second physical mark 24 provided on the case 21 of the ultrasonic probe 2 correspond with each other, referencing the orientation of the display mark on the VR image and the orientation of the second physical mark 24 enables the operator to easily determine a direction in which the ultrasonic probe 2 should be moved or rotated in order to obtain the desired image.

Incidentally, although the mark forming part 91 has written the straight mark 122 or the straight mark 123 into all the tomographic image data in this Example of Modification 2, it is possible to achieve the same effect and result even when writing the mark into a portion of tomographic image data.

In addition, the mark forming part 91 may write both marks of the straight mark 122 and mark 123 into the tomographic image data.

### Example of Modification 3

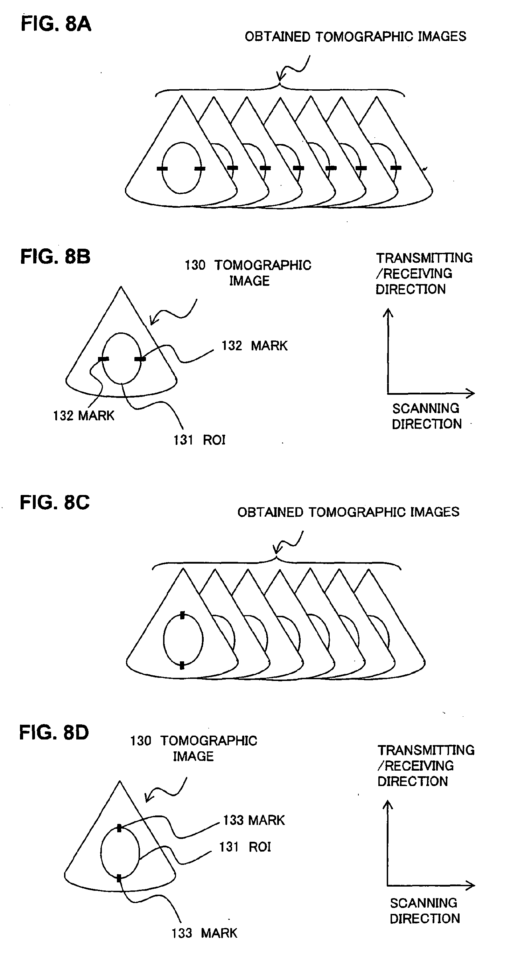

Next, Example of Modification 3 is described with reference to Fig. 8A, Fig. 8B, Fig. 8C, and Fig. 8D. Fig. 8A, Fig. 8B, Fig. 8C, and Fig. 8D are views of the processing for displaying a mark overlapping a three-dimensional image and a schematic drawing representing a tomographic image. As shown in Fig. 8A, the mark forming part 91 writes a mark into all tomographic image data obtained along the swing direction. As shown in Fig. 8B, for example, the mark forming part 91 writes a mark 132 into the left and right end parts of a preset ROI 131 for all tomographic image data 130. This mark 132 is written into the center of the transmitting/receiving direction in the ROI 131. For example, the mark forming part 91 colors the mark 132 or increases the pixel value thereof to be higher than that of the surrounding area. Information regarding the ROI 131 and information regarding the mark 132 are pre-stored in the condition storage 10. Then, the mark forming part 91 outputs all the tomographic image data into the end part of which the mark 132 has been written to the VR processor 92.

In addition, as shown in Fig. 8C and Fig. 8D, the mark forming part 91 may also write the mark 133 into the top and bottom end parts of the preset ROI 131 in all the tomographic image data 130. This mark 133 is written into the center of the scanning direction in the ROI 131. For example, the mark forming part 91 colors the mark 133 or increases the pixel value thereof to be higher than that of the surrounding area. Information regarding the mark 133 is pre-stored in the condition storage 10. Then, the mark forming part 91 outputs all the tomographic image data into the end part of which the mark 133 has been written to the VR processor 92.

The VR processor 92 receives the plurality of tomographic image data from the mark forming part 91, and applies volume rendering to generate the VR image data. The display 11 displays a VR image based on the VR image data (three-dimensional image) on the screen.

As described above, a mark is written into a plurality of tomographic image data and three-dimensional image data is generated based on the plurality of tomographic image data. As a result, a display mark corresponding to the mark is displayed on the VR image displayed on the display 11.

Since the mark 133 is written into the center of the scanning direction and the second physical mark 24 is provided at the center of the scanning direction of the case 21, the display mark on the VR image displayed on the display 11 corresponds with the second physical mark 24. Since the display mark on the VR image displayed on the display 11 and the second physical mark 24 provided on the case 21 of the ultrasonic probe 2 correspond with each other, referencing the orientation of the display mark on the VR image and the orientation of the second physical mark 24 enables the operator to easily determine in which direction the ultrasonic probe 2 should be moved or rotated in order to obtain the desired image.

Incidentally, although the mark forming part 91 has written the mark 132 or the mark 133 into all the tomographic image data in this Example of Modification 3, it is possible to achieve the same effect and result even when writing the mark into a portion of tomographic image data. For example, as in the above embodiment, the mark may be written into one tomographic image data.

The mark forming part 91 and the VR processor 92 described above may be implemented by hardware or software. For example, the calculator 9 may be implemented by a storage device such as a CPU (Central Processing Unit), a ROM (Read Only Memory), or a RAM (Random Access Memory). An image-processing program for performing the functions of the calculator 9 is stored on the storage device. This image-processing program includes a mark-forming program for performing the functions of the mark forming part 91, and a VR processing program for performing the functions of the VR processor 92. The CPU writes a mark into tomographic image data by performing the mark-forming program. In addition, the CPU performs volume rendering by performing the VR processing program.

### Operation

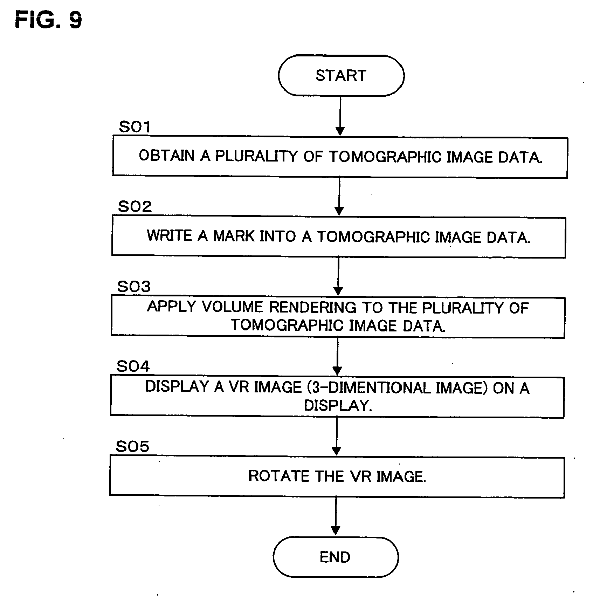

Next, a series of operations by the ultrasonic imaging apparatus 1 according to an embodiment of the present invention is described with reference to Fig. 9. Fig. 9 is a flow chart showing a series of operations by the ultrasonic imaging apparatus according to an embodiment of the present invention.

### Step S01

First, ultrasonic waves are transmitted to a subject to be examined using an ultrasonic probe 2, and a plurality of tomographic image data is obtained, based on reflected waves from the subject to be examined. Herein, by employing a one-dimensional array probe as the ultrasonic probe 2 and transmitting/receiving ultrasonic waves while swinging ultrasonic transducers in the direction perpendicular to the scanning direction (swing direction), a plurality of tomographic image data along the swing direction is obtained. The plurality of tomographic image data is stored on the storage 8.

### Step S02

Next, the calculator 9 reads the plurality of tomographic image data along the swing direction from the storage 8. Then, the mark forming part 91 selects tomographic image data at a predefined position among the plurality of tomographic image data, and writes a predetermined mark into the tomographic image data. For example, as shown in Fig. 5A, the mark forming part 91 selects tomographic image data obtained at the center of the swing direction among a plurality of tomographic image data obtained along the swing direction. Then, as shown in Fig. 5B, the mark forming part 91 colors images included in a preset ROI 101 with a preset color for the tomographic image data 100 obtained at the center of the swing direction. Then, the mark forming part 91 sends a plurality of tomographic image data including the colored tomographic image data to the VR processor 92.

The center of the swing direction corresponds with the position of the first physical mark 23 provided at the center of the swing direction of the case 21. That is, since the mark is written into the tomographic image data that has been acquired at the center of the swing direction and the first physical mark 23 is provided at the center of the swing direction of the case 21, the position of the mark written into the tomographic image data and the position of the first physical mark 23 correspond with each other.

### Step S03

Next, the VR processor 92 generates volume data by means of a known method, based on the plurality of tomographic image data, and applies volume rendering to the volume data to generate three-dimensional image data (VR image data). At this time, the VR processor 92 generates VR image data seen from a predetermined direction by performing volume rendering along a preset eye-gaze direction. The VR processor 92 outputs the VR image data to the display 11.

### Step S04

Upon receiving the VR image data from the VR processor 92, the display 11 displays a VR image based on the VR image data on the screen. A display mark, which corresponds to the mark written into the tomographic image data at Step S02, is displayed on the VR image displayed on the display 11.

Since a mark is written into the tomographic image data obtained at the center of the swing direction and the first physical mark 23 is provided at the center of the swing direction of the case 21, the display mark on the VR image displayed on the display 11 corresponds with the first physical mark 23. Consequently, referencing the orientation of the display mark on the VR image and the orientation of the first physical mark 23 enables the operator to easily determine a direction in which the ultrasonic probe 2 should be moved or rotated in order to obtain the desired image.

Incidentally, at Step S02, any mark of Examples of Modification 1 through 3 described above may be formed instead of the marks shown in Fig. 5A and Fig. 5B. For example, as in Example of Modification 1 shown in Fig. 6A and Fig. 6B, a frame 112 surrounding a preset ROI 111 may be written as a mark into the tomographic image data 110 obtained at the center of the swing direction. Since the center of the swing direction corresponds with the position of the first physical mark 23 provided at the center of the swing direction of the case 21, the position of the mark written into the tomographic image data and the position of the first physical mark 23 correspond with each other.

Since the frame (mark) 112 is written into the tomographic image data obtained at the center of the swing direction and the first physical mark 23 is provided at the center of the swing direction of the case 21, the display mark on the VR image displayed on the display 11 corresponds with the first physical mark 23. Consequently, referencing the orientation of the display mark on the VR image and the orientation of the first physical mark 23 enables the operator to easily determine a direction in which the ultrasonic probe 2 should be moved or rotated in order to obtain the desired image.

In addition, as in Example of Modification 2 shown in Fig. 7A through Fig.7D, the mark may be written into all the tomographic image data 120. For example, as shown in Fig. 7B, the mark forming part 91 writes the straight mark 122 crossing in the scanning direction (transverse direction) at the center of the ROI 121 into all tomographic image data 120. In addition, as shown in Fig. 7C, the mark forming part 91 writes the straight mark 123 expanding in the transmitting/receiving direction (longitudinal direction) at the center of the scanning direction of the ROI 121 into all tomographic image data 120. The center of the scanning direction corresponds with the position of the second physical mark 24 provided at the center of the scanning direction of the case 21. That is, since the straight mark 123 is written into the center of the scanning direction and the second physical mark 24 is provided at the center of the scanning direction of the case 21, the position of the mark written into the tomographic image data and the position of the second physical mark 24 correspond with each other.

Since the straight mark 123 is written into the center of the scanning direction and the second physical mark 24 is provided at the center of the scanning direction of the case 21, the display mark on the VR image displayed on the display 11 corresponds with the second physical mark 24. Consequently, referencing the orientation of the display mark on the VR image and the orientation of the second physical mark 24 enables the operator to easily determine a direction in which the ultrasonic probe 2 should be moved or rotated in order to obtain the desired image.

In addition, as in Example of Modification 3 shown in Fig. 8A through Fig. 8D, the mark may be written into all the tomographic image data 130. For example, as shown in Fig. 8B and Fig. 8D, the mark forming part 91 writes the mark 132 or the mark 133 into the end part of the ROI 131 for all the tomographic image data 130. The mark 132 is written into the center of the transmitting/receiving direction in the ROI 131. The mark 133 is written into the center of the scanning direction in the ROI 131. The center of the scanning direction corresponds with the position of the second physical mark 24 provided at the center of the scanning direction of the case 21. That is, since the mark 133 is written into the center of the scanning direction and the second physical mark 24 is provided at the center of the scanning direction of the case 21, the position of the mark written into the tomographic image data and the position of the second physical mark 24 correspond with each other.

Since the mark 133 is written into the center of the scanning direction and the second physical mark 24 is provided at the center of the scanning direction of the case 21, the display mark on the VR image displayed on the display 101 corresponds with the second physical mark 24. Consequently, referencing the orientation of the display mark on the VR image and the orientation of the second physical mark 24 enables the operator to easily determine a direction in which the ultrasonic probe 2 should be moved or rotated in order to obtain the desired image.

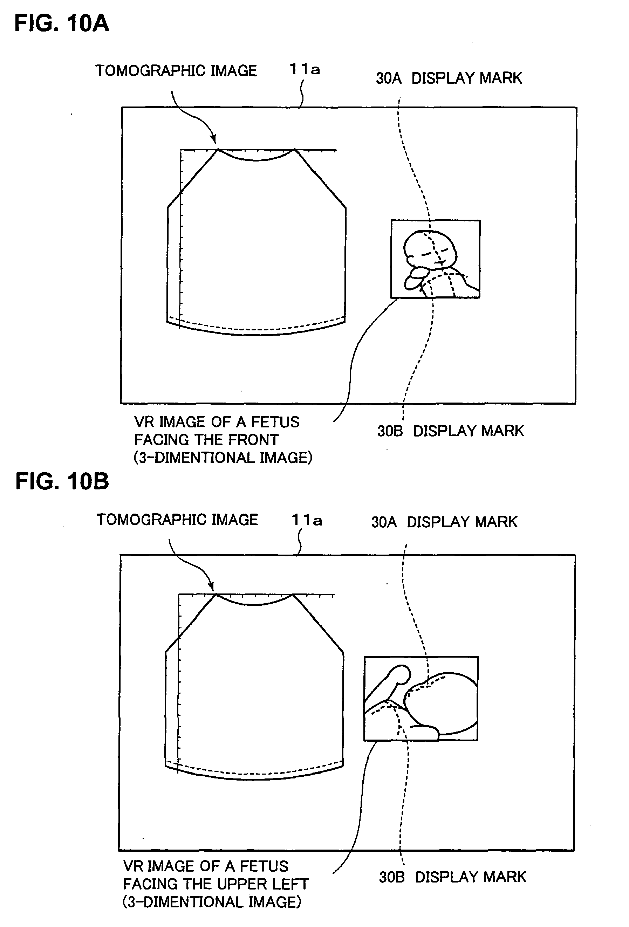

Herein, examples of the display of the VR image and the display mark are shown in Fig. 10A and Fig. 10B. Fig. 10A and Fig. 10B are views of a screen showing a three-dimensional image obtained by the ultrasonic imaging apparatus according to an embodiment of the present invention. For example, as shown in Fig. 10A, a three-dimensional image of a fetus is displayed on the screen 11 a of the display 10. In the example shown in Fig. 10A, the three-dimensional image of the fetus is facing the front. The display marks 30A and 30B are displayed overlapping this three-dimensional image of the fetus. These display marks 30A and 30B are the display of the marks written into predetermined tomographic image data at Step S02. For example, the display mark 30A corresponds with a mark written into the center of the swing direction in the tomographic image data, and the display mark 30B corresponds with a mark written into the center of the scanning direction in the tomographic image data.

### Step S05

As shown in Fig. 10A, the VR image is displayed on the display 11. When the operator uses an operating part (not shown) to give instructions to rotate a VR image, the VR processor 92 applies volume rendering from a different eye-gaze direction upon receiving the rotating instructions. Consequently, a VR image to be seen from a different direction can be obtained. For example, as shown in Fig. 10B, it is possible to display the three-dimensional image of the fetus on the screen 11 a such that it is facing the upper left.

The positional relationship is clear between the display mark 30A or the display mark 30B displayed on the VR image and the first physical mark 23 or the second physical mark 24 provided on the ultrasonic probe 2. This enables the direction in which the ultrasonic probe 2 should be moved or rotated in order to obtain the desired image to be easily determined, thereby making it possible to improve operability of the ultrasonic probe 2.

In addition, the display mark may be capable of switching between display/hide. For example, when the first physical mark 23 or the second physical mark 24 provided on the ultrasonic probe 2 is used as a changeover switch, and the first physical mark 23 or the second physical mark 24 is pressed, the mark forming part 91 writes the mark into a predetermined tomographic image data so as to display the mark on the VR image. In addition, when the first physical mark 23 or the second physical mark 24 is pressed while the mark is displayed on the VR image, the mark forming part 91 ceases writing the mark into the tomographic image data so as not to display the mark on the VR image.

For example, the display mark is displayed on the VR image when moving or rotating the ultrasonic probe 2. Referencing the display mark and the first physical mark 23 or the second physical mark 24 provided on the ultrasonic probe 2 enables the operator to easily determine the direction in which the ultrasonic probe 2 will be moved or rotated. On the other hand, when there is no need to move or rotate the ultrasonic probe 2, it is possible to display only the VR image, without displaying the display mark, to observe the VR image in detail.

Incidentally, in the embodiments and examples of modification described above, a one-dimensional array probe has been employed as the ultrasonic probe 2. A two-dimensional array probe may also be employed instead of the one-dimensional array probe. In this case, it is possible to achieve the same effect and result as the embodiments and examples of modification described above by forming a mark on the three-dimensional image data obtained by the two-dimensional array probe and displaying the three-dimensional image.

For example, when obtaining three-dimensional volume data by employing the two-dimensional array probe, the mark forming part 91 writes a mark for indicating the positional relationship with the ultrasonic probe into a predetermined position on the volume data. Then, the VR processor 92 applies volume rendering to the volume data to generate the VR image data. Writing a mark into a predetermined position on volume data and generating three-dimensional image data based on the volume data results in a display mark corresponding to the mark written into the predetermined position being displayed on the VR image displayed on the display 11. Referencing this mark enables the relative positional relationship between the ultrasonic probe 2 and the three-dimensional image to be easily ascertained.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. An ultrasonic imaging apparatus comprising:
an ultrasonic probe configured to transmit ultrasonic waves to a subject to be examined and to receive the reflected waves from said subject to be examined, and
an image processor configured to generate three-dimensional image data based on the reflected waves received by said ultrasonic probe, and to instruct a display to display a mark indicating the positional relationship between the three-dimensional image and said ultrasonic probe, said mark overlapping said three-dimensional image based on said three-dimensional image data.

2. An ultrasonic imaging apparatus according to Claim 1, wherein
said image processor adds the mark indicating the positional relationship between said three-dimensional image and said ultrasonic probe to said three-dimensional image data, and instructs said displays to display a three-dimensional image based on the three-dimensional image data to which said mark has been added.

3. An ultrasonic imaging apparatus according to Claim 2, wherein
said image processor writes said mark into a preset position in said three-dimensional image data.

4. An ultrasonic imaging apparatus according to Claim 1, wherein
said image processor displays at least one mark from a linear mark along the transmitting/receiving direction of said ultrasonic waves and a liner mark along the direction perpendicular to said transmitting/receiving direction on said display, said linear marks overlapping the three-dimensional image based on said three-dimensional image data.

5. An ultrasonic imaging apparatus according to Claim 1, wherein
said image processor comprises:
a tomographic image data generator configured to generate a plurality of tomographic image data along a predetermined direction based on the reflected waves received by said ultrasonic probe,
a mark forming part configured to write a predetermined mark into tomographic image data obtained at a predefined position in said predetermined direction among the plurality of tomographic image data along said predetermined direction, and
a three-dimensional image data generator configured to generate three-dimensional image data based on said plurality of tomographic image data including the tomographic image data into which said predetermined mark has been written,
and wherein
said image processor instructs said display to display a three-dimensional image based on the three-dimensional image data generated by said three-dimensional image data.

6. An ultrasonic imaging apparatus according to Claim 5, wherein
said ultrasonic probe comprises a plurality of ultrasonic transducers arranged in a line and a case housing said plurality of ultrasonic transducers, wherein said ultrasonic probe is configured to transmit the ultrasonic waves to said subject to be examined and to receive the reflected waves from said subject to be examined, using the direction of said arrangement as the scanning direction of the ultrasonic waves, while swinging said plurality of ultrasonic transducers in the swing direction perpendicular to the direction of said arrangement,
said tomographic image data generator generates a plurality of tomographic image data along said swing direction based on the reflected waves received by said ultrasonic probe, and
said mark forming part writes said predetermined mark into tomographic image data obtained at a predefined position in said swing direction among the plurality of tomographic image data along said swing direction.

7. An ultrasonic imaging apparatus according to Claim 6, wherein
a first physical mark for specifying the predefined position in said swing direction is provided on the external surface of said case.

8. An ultrasonic imaging apparatus according to Claim 7, wherein
said first physical mark is provided on the external surface parallel to said swing direction of said case.

9. An ultrasonic imaging apparatus according to Claim 5, wherein
said mark forming part colors the tomographic image data obtained at said predefined position with a predetermined color, and
said three-dimensional image data generator generates three-dimensional image data based on a plurality of tomographic image data including said colored tomographic image data.

10. An ultrasonic imaging apparatus according to Claim 5, wherein
said mark forming part colors a predetermined range of the tomographic image data obtained at said predefined position with a predetermined color, and
said three-dimensional image data generator generates three-dimensional image data based on a plurality of tomographic image data including said colored tomographic image data.

11. An ultrasonic imaging apparatus according to Claim 5, wherein
said mark forming part writes, as said predetermined mark, a frame surrounding the predetermined range of the tomographic image data obtained at said predefined position.

12. An ultrasonic imaging apparatus according to Claim 5, wherein
said mark forming part writes said predetermined mark into a portion of the boundary of the predetermined range of the tomographic image data obtained at said predefined position.

13. An ultrasonic imaging apparatus according to Claim 6, wherein
said mark forming part writes said predetermined mark into tomographic image data obtained at the general center of said swing direction.

14. An ultrasonic imaging apparatus according to Claim 13, wherein
a first physical mark is formed on the external surface parallel to said swing direction of said case that is at the general center of said swing direction.

15. An ultrasonic imaging apparatus according to Claim 1, wherein
said image processor comprises:
a tomographic image data generator configured to generate a plurality of tomographic image data along a predetermined direction based on the reflected waves received by said ultrasonic probe,
a mark forming part configured to write a predetermined mark into a predetermined range of each tomographic image data for the plurality of tomographic image data along said predetermined direction, and
a three-dimensional image data generator configured to generate three-dimensional image data based on said plurality of tomographic image data into which said predetermined mark has been written, and
wherein said image processor instructs a display to display a three-dimensional image based on the three-dimensional image data generated by said three-dimensional image data generator.

16. An ultrasonic imaging apparatus according to Claim 15, wherein
said ultrasonic probe comprises a plurality of ultrasonic transducers arranged in a line and a case housing said plurality of ultrasonic transducers, wherein said ultrasonic probe is configured to transmit the ultrasonic waves to said subject to be examined and to receive the reflected waves from said subject to be examined, using the direction of said arrangement as the scanning direction of the ultrasonic waves, while swinging said plurality of ultrasonic transducers in the swing direction perpendicular to the direction of said arrangement,
said tomographic image data generator generates a plurality of tomographic image data along said swing direction based on the reflected waves received by said ultrasonic probe, and
said mark forming part writes a predetermined mark into a predetermined range of each tomographic image data for the plurality of tomographic image data along said swing direction.

17. An ultrasonic imaging apparatus according to Claim 16, wherein
said mark forming part writes at least one mark from a linear mark along a transmitting/receiving direction of said ultrasonic waves and a linear mark along said scanning direction into a predefined position in each tomographic image data for the plurality of tomographic image data along said swing direction.

18. An ultrasonic imaging apparatus according to Claim 17, wherein
a second physical mark for specifying said predefined position is formed on the external surface of said case.

19. An ultrasonic imaging apparatus according to Claim 18, wherein
said second physical mark is formed on the external surface parallel to said scanning direction of said case.

20. An ultrasonic imaging apparatus according to Claim 15, wherein
said mark forming part writes said predetermined mark into a portion of the boundary of a predetermined range of each tomographic image data for the plurality of tomographic image data along said predetermined direction.

21. A method of displaying ultrasonic images comprising:
transmitting ultrasonic waves to a subject to be examined and receiving the reflected waves from said subject to be examined using an ultrasonic probe, and
generating three-dimensional image data based on the reflected waves received by said ultrasonic probe to display a mark indicating the positional relationship between the three-dimensional image and said ultrasonic probe on a display, said mark overlapping said three-dimensional image based on said three-dimensional image data.

22. A method of displaying ultrasonic images according to Claim 21, wherein said step of generation comprises:
generating a plurality of tomographic image data along a predetermined direction based on the reflected waves received by said ultrasonic probe,
writing a predetermined mark into tomographic image data obtained at a predefined position in said predetermined direction among the plurality of tomographic image data along said predetermined direction,
generating three-dimensional image data based on said plurality of tomographic image data including the tomographic image data into which said predetermined mark has been written, and
displaying a three-dimensional image based on said three-dimensional image data on a display.

23. A method of displaying ultrasonic images according to Claim 21, wherein
said step of generation comprises:
generating a plurality of tomographic image data along a predetermined direction based on the reflected waves received by said ultrasonic probe,
writing a predetermined mark into a predetermined range of each tomographic image data for the plurality of tomographic image data along said predetermined direction,
generating three-dimensional image data based on the plurality of tomographic image data into which said predetermined mark has been written, and
displaying a three-dimensional image based on said three-dimensional image data on a display.
